Europäisches Patentamt

European Patent Office

Office européen des brevets

(19)

(11) Veröffentlichungsnummer : **0 178 514**
**B1**

(12) **EUROPÄISCHE PATENTSCHRIFT**

(45) Veröffentlichungstag der Patentschrift :
30.11.88

(21) Anmeldenummer : **85112230.9**

(22) Anmeldetag : **26.09.85**

(51) Int. Cl.⁴ : **A 61 N   1/04**

(54) **Bipolarer Stimulator zur Inkontinenzbehandlung durch Elektrostimulation.**

(30) Priorität : **13.10.84 DE 8430165 U**

(43) Veröffentlichungstag der Anmeldung :
**23.04.86 Patentblatt 86/17**

(45) Bekanntmachung des Hinweises auf die Patenterteilung : **30.11.88 Patentblatt 88/48**

(84) Benannte Vertragsstaaten :
**AT BE CH DE FR GB IT LI NL SE**

(56) Entgegenhaltungen :
**DE-A- 2 502 620
DE-A- 2 822 616
DE-A- 3 306 037
FR-A- 2 348 712
US-A- 2 085 644
US-A- 3 650 275
US-A- 3 749 100
US-A- 3 800 800
US-A- 3 943 938**

(73) Patentinhaber : **Rowedder, Klaus, Dr.
Tannenweg 6
D-2352 Mühbrook (DE)**

(72) Erfinder : **Rowedder, Klaus, Dr.
Tannenweg 6
D-2352 Mühbrook (DE)**

(74) Vertreter : **Wilcken, Thomas, Dipl.-Ing. et al
Musterbahn 1
D-2400 Lübeck (DE)**

EP 0 178 514 B1

**Beschreibung**

Die Erfindung betrifft einen bipolaren Stimulator zur Inkontinenzbehandlung des Mastdarm-Schließmuskels und der Beckenbodenmuskulatur durch Elektrostimulation, bestehend aus einem Teller zur Begrenzung der Einführtiefe und aus einem Kopf, der über einen Schaft dünneren Durchmessers mit dem Teller verbunden ist.

Die Inkontinenzbehandlung mit Reizströmen hat sich in der Praxis gut bewährt, auch wenn positive Behandlungsergebnisse nur bei Erfüllung bestimmter Voraussetzungen erreichbar sind. Insbesondere sind auch eine zweckmäßige Form und Art der Elektroden von entscheidender Bedeutung für den Behandlungserfolg.

Hinsichtlich der Elektrodenart und Elektrodenzahl unterscheidet man zwischen monopolaren und bipolaren Stimulatoren. Solche Stimulatoren sind beispielsweise dargestellt und beschrieben im deutschen Gebrauchsmuster 82 28 852.6, in der deutschen Offenlegungsschrift 33 06 037 und in den US-Patenten 3 403 684, 3 749 100, 3 800 800 und 3 933 147.

Bei der Anwendung monopolarer Elektroden bzw. Stimulatoren benötigt man außer der in den Schließmuskelbereich einzuführenden Elektrode zusätzlich eine weitere, meist großflächig ausgebildete Gegenelektrode, die extern am Rücken- oder Beckenbereich des Patienten anzubringen ist. Die einzuführende Elektrode kann beispielsweise aus einem im wesentlichen ovalen oder eiförmigen Kopf und einem Teller bestehen, die über einen Schaft verbunden sind. Kopf, Schaft und Teller bilden einheitlich eine Metallelektrode, die relativ schwer ist, falls nicht nur die Oberfläche der Elektrode durch einen Belag elektrisch leitend gemacht wird. Solche Elektroden sind beispielsweise im schon erwähnten deutschen Gebrauchsmuster dargestellt und beschrieben.

Die Handhabung und die Befestigung der außerdem erforderlichen Gegenelektroden bringen einen relativ großen Aufwand mit sich, zumal diese Elektrode meist mit einer Bandage in ihrer Position festgelegt werden muß. Häufig ist auch nicht zu vermeiden, daß die Gegenelektrode vor allem bei Anwendung relativ hoher Reizströme Schmerzen auf der Haut verursacht und die Haut im übrigen unnötig stimuliert bzw. gereizt wird.

Besser sind insofern bipolare Stimulatoren, bei denen zwei gegeneinander elektrisch isolierte Elektroden mit Abstand zueinander an einem Kunststoffträger angebracht sind und mit den beiden Polen einer Reizstromquelle in Verbindung stehen. Die beiden Metallelektroden bekannter Stimulatoren haben die Form von Ringen, Rohrstücken oder Streifen aus Metall, die dünn, kurz oder schmal sind und somit eine relativ kleine wirksame Oberfläche für den Stromübergang zum Körper des Patienten haben. Dies hat zur Folge, daß das Stromfeld bzw. die Stromverteilung im Bereich der Elektroden inhomogen ist und daß regional ggf. auch so hohe Stromdichten entstehen können, daß die Schmerzschwelle erreicht und überschritten wird. Das gilt weniger für bipolare Stimulatoren, deren Elektroden eine relativ große Oberfläche haben. Insofern sind also Stimulatoren zweckmäßig, die im französischen Patent 74 20 974 dargestellt sind und bei denen die distale Elektrode einen Teil des Kopfes und die andere Elektrode einen Teil des proximalen « Tellers » abdeckt, so daß vergleichsweise große Elektrodenflächen entstehen, die von einem Schaft aus Isoliermaterial getrennt sind.

Die Praxis hat aber gezeigt, daß auch solche Stimulatoren vor allem bei Anwendung von hohen Reizströmen nicht befriedigen können. Insbesondere ist ein entscheidender Nachteil darin zu sehen, daß sich nur ein begrenztes Stromfeld ringförmig um die Längsachse des Stimulators ausbilden kann. Dieses Stromfeld wird also nur die Nerven des Schließmuskels, nicht jedoch die Nerven der tiefer im Beckenboden liegenden Muskulatur erreichen.

Die Aufgabe der Erfindung besteht in der Beseitigung der aufgezeigten Nachteile. Es soll also ein Stimulator zur Inkontinenzbehandlung vorgeschlagen werden, der einfach und sicher in seiner Anwendung ist und der gegenüber den bekannten Stimulatoren eine effektivere Stimulation selbst bei schwierigen Behandlungsfällen gewährleistet.

Zur Lösung dieser Aufgabe wird der eingangs erwähnte Stimulator so ausgebildet, daß der gesamte Kopf und zumindest der am Schaft angrenzende Teil des Tellers elektrisch leitend sind und zwei Elektroden bilden, die durch den Schaft elektrisch gegeneinander isoliert sind.

Stimulatoren mit einem im Prinzip eiförmigen Kopf und einem hiermit über einen zylindrischen Schaft verbundenen Teller haben sich schon hinsichtlich ihrer Form als monopolare Instrumente bewährt. Die im Zusammenhang mit der praktischen Anwendung gegebenen Vorteile solcher Monopolarinstrumente können auch voll beim bipolaren Instrument bzw. Stimulator nach der vorliegenden Erfindung zur Geltung kommen, während darüber hinaus eine externe Gegenelektrode entfällt und die zwangsläufig relativ großen Flächen der beiden durch den Kopf einerseits und den Teller andererseits gebildeten Elektroden dazu beitragen, daß selbst dann, wenn die den Elektroden zugeführten Ströme hoch sind, die wirksame Stromdichte an den Elektrodenflächen ungefährlich ist und trotzdem eine einwandfreie Stimulation der zu behandelnden Körperbereiche erfolgt. Da der Kopf insgesamt als leitende Elektrode dient, wird sich aufgrund der Ball-, Ei- oder Ovalform des Kopfes eine Stromverteilung ergeben, die auch Nerven von tiefer liegenden Muskeln im Beckenboden erreicht und stimulieren kann, was mit allen bisher bekannten monopolaren und bipolaren Stimulatoren nicht oder höchstens dann möglich war, wenn man sehr hohe und damit gefährliche Stimulationsströme angewendet hat.

Der hohlzylindrisch oder rohrförmig ausgebildete Schaft wird zweckmäßigerweise in seinem Durchgang mit einem Innengewinde versehen, während der Teller und der Kopf mit Gewindestutzen ausgestattet sind, so daß einer der Gewindestutzen in das eine Ende und der andere Gewindestutzen in das andere Ende des Durchganges geschraubt werden können. Auf diese Weise wird eine besonders einfache, sichere und elektrisch isolierende Verbindung zwischen den beiden metallischen, durch Kopf und Teller gebildeten Elektroden geschaffen.

Im übrigen sollte ein Dichtungsmittel im Bereich der Schraubverbindungen und/oder im Bereich der gegenseitigen Stoßstellen zwischen dem Schaft einerseits und dem Kopf sowie Teller andererseits vorgesehen werden, so daß bei der Anwendung und Reinigung des Stimulators keine Feuchtigkeit oder Schmutz in das Innere des Stimulators dringen kann.

Nachfolgend wird das auf der anliegenden Zeichnung schematisch und teilweise im Schnitt dargestellte Ausführungsbeispiel der Erfindung beschrieben.

Das distale Ende des Instrumentes wird durch den Kopf 1 gebildet, der eine eiovale Form hat und über den Schaft 2 aus Isoliermaterial mit dem Teller 3 verbunden ist. Der Kopf und der Teller bestehen aus leitendem Metall, wie z. B. V4A-Stahl, und bilden beide Elektroden des Stimulators. Es wäre auch möglich, daß nur die Oberflächenbereiche des Kopfes und des Tellers elektrisch leitend sind, was man einfach dadurch erreichen kann, daß entsprechend geformte Träger aus Isolierstoff metallisch beschichtet werden.

Weiterhin kann es genügen, wenn nur der an den Schaft 2 angrenzende und nach der Darstellung kegelstumpfförmige Teil der Elektrode 3 elektrisch leitend ist, obwohl voraussichtlich eine bessere homogene Stromverteilung erreicht wird, wenn der gesamte Teller aus Metall besteht bzw. mit Metall beschichtet ist. Außerdem ist es möglich, daß die beiden Elektroden auf den Schaft übergehen, so daß nur ein entsprechend kürzerer Teil des Schaftes aus Isoliermaterial bestehen wird, während die Schaftenden leitend sind und Teile der Elektroden bilden.

· Der Schaft 2 ist als Rohr bzw. Hohlzylinder ausgebildet, dessen Durchgang 2a mit Gewinde versehen ist. Der Kopf 1 ist mit einem Gewindestutzen 1a vom einen Ende her und der Teller 3 mit einem Gewindestutzen 3a vom anderen Ende her in den Durchgang 2a geschraubt.

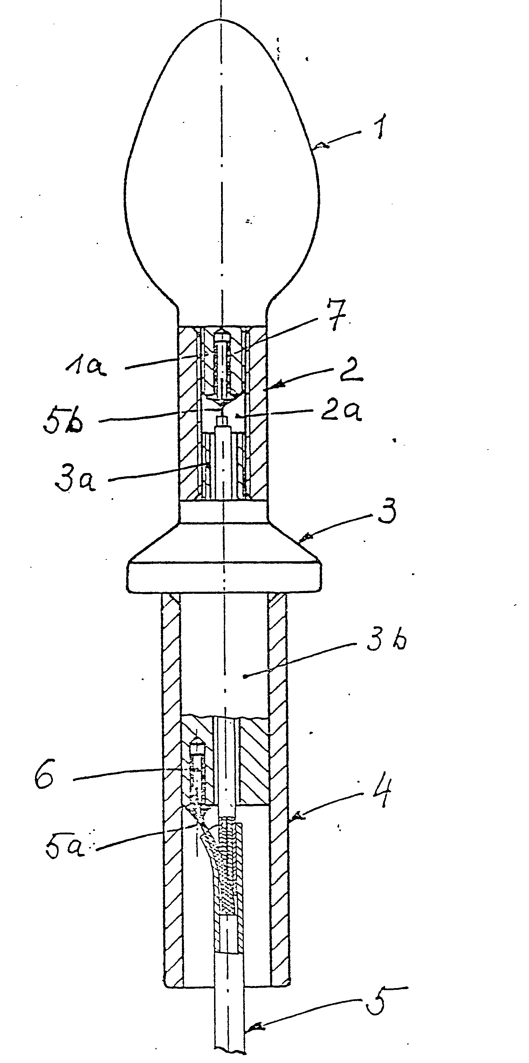

Wie bereits erwähnt wurde, sollte im Bereich dieser Schraubverbindungen ein Dichtungsmittel vorgesehen werden. Zusätzlich oder stattdessen soll auch im Bereich der gegenseitigen Stoßstellen zwischen dem Schaft 2 einerseits und dem Kopf 1 sowie Teller 3 andererseits ein Dichtungsmittel vorgesehen werden, wobei als Dichtungsmittel beispielsweise ein Kleber oder Kunstkautschuk zur Anwendung kommen kann, der einen dünnen und deshalb in der Zeichnung nicht sichtbaren Film oder Belag im erwähnten Verbindungsbereich bildet.

Der Elektrodenteller 3 greift mit einem Fortsatz 3b in eine Hülse 4, die aus Isoliermaterial besteht und in an sich bekannter Weise u. a. als Handhabe für den Stimulator dient. In diese Hülse ist von außen das von einem nicht gezeigten Stimulationsgerät kommende Kabel 5 eingeführt. Ein Kabelleiter 5a ist elektrisch mit dem Fortsatz 3b verbunden, während ein weiterer Kabelleiter 5b isoliert durch eine Längsbohrung der Elektrode 3 verläuft und elektrisch mit dem Gewindestutzen 1a verbunden ist.

Die Verbindung der den regelbaren Reizstrom führenden Leiter mit den beiden Elektroden 1, 3 erfolgt mit Hilfe von üblichen Klemmkontakten 6, 7, die in Sacklöchern der Teile 1a, 3b stecken und die freien Enden der jeweiligen Kabelleiter aufnehmen.

Die praktische Handhabung und die grundsätzliche Funktion des erfindungsgemäßen Stimulators sind im Prinzip bekannt und sollen deshalb nicht im einzelnen beschrieben werden. Soweit die Handhabung und das Einführen des Stimulators betroffen sind, kann insofern auf die einen monopolaren Stimulator betreffende deutsche Offenlegungsschrift 33 06 037 hingewiesen werden. Im übrigen sind die Funktionen und Betriebsweisen bipolarer Stimulatoren u. a. den schon einleitend erwähnten US-Patenten zu entnehmen.

## Patentansprüche

1. Bipolarer Stimulator zur Inkontinenzbehandlung des Mastdarm-Schließmuskels und der Beckenbodenmuskulatur durch Elektrostimulation, bestehend aus einem ball- oder eiförmigen Kopf, aus einem die Einführtiefe begrenzenden Teller, aus einem im Durchmesser kleineren, aus isolierendem Werkstoff hergestellten Schaft mit einem axialen Durchführungskanal und aus zwei axial voneinander beabstandeten, durch das Schaftmaterial gegeneinander isolierten Elektroden, wobei das eine Schaftende den Kopf und das andere Schaftende den Teller trägt, dadurch gekennzeichnet, daß in an sich bekannter Weise der gesamte Kopf (1) und zumindest der an den Schaft (2) angrenzende Teil des Tellers (3) elektrisch leitend sind und daß der Schaft (2) über seine gesamte Länge nach außen isolierend ist.

2. Stimulator nach Anspruch 1, dadurch gekennzeichnet, daß der rohrförmige Schaft (2) in seinem Durchführungskanal (2a) mit Innengewinde versehen ist und daß der Kopf (1) und der Teller (3) Gewindestutzen (1a, 3a) aufweisen, wobei der eine Gewindestutzen in das eine Ende und der andere Gewindestutzen in das andere Ende des Durchführungskanals geschraubt ist.

3. Stimulator nach Anspruch 2, dadurch gekennzeichnet, daß Dichtungsmittel im Bereich der Schraubverbindungen und/oder im Bereich der gegenseitigen Stoßstellen zwischen dem Schaft (2) einerseits und dem Kopf (1) sowie dem Teller (3) andererseits vorgesehen sind.

## Claims

1. Bipolar stimulator for treatment of incontinence of the rectal sphincter and of the pelvic floor musculature by electric stimulation, comprising a ball-shaped or egg-shaped head, a plate limiting the depth of insertion, a shaft of smaller diameter formed from insulating material and having an axial lead-through passage, and two electrodes axially spaced apart one from the other and insulated one from another by the shaft material, the one shaft extremity carrying the head and the other shaft extremity carrying the plate, characterised in that in a manner known per se, the entire head (1) and at least the part of the plate (3) Adjacent to the shaft (2) are electrically conductive and that the shaft (2) is outwardly insulating throughout its length.

2. Stimulator according to claim 1, characterised in that the tubular shaft (2) is provided with an internal screw-thread in its lead-through passage (2a) and that the head (1) and the plate (3) have screw-threaded connectors (1a, 3a), the one screw-threaded connector being screwed into the one end of the lead-through passage and the other screw-threaded connector being screwed into its other end.

3. Stimulator according to claim 2, characterised in that sealing means are provided in the area of the screw connections and/or in the areas of the points of mutually abutting contact between the shaft (2) on the one hand and the head (1) as well as the plate (3) on the other hand.

**Revendications**

1. Stimulateur bipolaire pour le traitement d'incontinences du sphincter du rectum et des muscles du plancher pelvien, par stimulation électrique, constitué par une tête en forme de ballon ou d'oeuf, un disque limitant la profondeur de pénétration, une tige d'un diamètre plus faible, réalisée en un matériau isolant et possédant un canal traversant axial, et deux électrodes espacées axialement et isolées l'une par rapport à l'autre par le matériau de la tige, une extrémité de la tige portant la tête et l'autre extrémité le disque, caractérisé en ce que, d'une façon connue en soi, l'ensemble de la tête (1) et au moins la partie du disque (3), jouxtant la tige (2) sont électriquement conducteurs et en ce que la tige (2) est isolante en direction de l'extérieur sur toute sa longueur.

2. Stimulateur selon la revendication 1, caractérisé en ce que la tige tubulaire (2) comporte un taraudage dans son canal traversant (2a) et en ce que la tête (1) et le disque (3) possèdent des embouts filetés (1a, 3a), un embout fileté étant vissé dans une extrémité du canal traversant et l'autre embout fileté étant vissé dans l'autre extrémité du canal traversant.

3. Stimulateur selon la revendication 2, caractérisé en ce que des moyens d'étanchéité sont prévus dans la zone de liaison vissée et/ou dans la zone des points de contact réciproques entre la tige (2) d'une part et la tête (1) ainsi que le disque (3) d'autre part.

0 178 514